# EUROPEAN PATENT APPLICATION

(11) **EP 4 847 060 A1**
(43) Date of publication of application: **02.12.2026**
(21) Application number: 25179176.0
(22) Date of filing: 27.05.2025
(51) Int. Cl.: A61M 5/145, A61M 5/315, A61M 5/24

(54) **ACTUATION OF RESET MECHANISM OF DRUG DELIVERY DEVICE**

(71) Applicant: Ypsomed AG, 3401 Burgdorf (CH)
(72) Inventor: Kalbermatter, Gabriel, 3400 Burgdorf (CH); Bosshard, Simon Martin, 3324 Hindelbank (CH); Jenni, Mai, 4600 Olten (CH); Caniglia, Alessandro, 3400 Burgdorf (CH)
(74) Representative: Kleiner, Stefan

(57) **Abstract**

The invention relates to a drug delivery device (1) for dispensing a liquid drug from a reservoir (3), the delivery device (1) comprising a reservoir holder (30), collar (10) comprising a fastening element (11) fastening the collar (10) non-releasably to a neck portion of the reservoir (3). The collar further comprises a collar connecting portion (15) adapted to be releasably coupled to a holder connecting portion (45) of the reservoir holder. The delivery device further includes a coupling member (170), a rotatable plunger rod (160) and a reverse lock, wherein when the coupling member (170) is in a reset position the plunger rod (160) is decoupled from the reverse lock wherein when the coupling member (170) is in a drive position the plunger rod (160) is coupled to the reverse lock.

## Description

### FIELD OF THE INVENTION

The present invention relates to a drug delivery device for dispensing a liquid drug through an injection needle for injecting, delivering, administering or dispensing substances and/or liquids such as insulin, hormone preparations or vaccines. The delivery device comprises a reservoir holder, a collar with a connecting portion adapted to be releasably coupled to a reservoir holder connecting portion and a coupling member which can be selectively coupled to a reverse lock of the delivery device.

### BACKGROUND OF THE INVENTION

A variety of diseases exist that require regular treatment by subcutaneous administration of a medicament, and a number of drug delivery devices have been developed to support a patient in accurately and controllably delivering an amount of drug in a self-administration process. Delivery devices include injection devices that are removed from the injection site after each medication event or drug delivery process, as well as infusion devices with a cannula or needle that remains in the skin of the patient for a prolonged period of time.

By way of example, diabetes may be treated by self-administration of insulin or its derivatives with the help of multi-variable-dose insulin injection pens. An injection pen device generally has an elongate device body defining a longitudinal main device axis. An automatic injection device has a motor or a drive spring for biasing a plunger rod and shifting a piston in a container barrel, wherein the drive spring may have to be charged or strained manually prior to injection of a dose. A manually powered delivery drive requires a user to manually provide the energy to move the piston, for instance by applying a distal force component to the injection device.

The medicament dose to be injected may be manually selected by turning a dosage knob and observing the actual dialed dose from a dose window or display of the injection pen. A dose is dispensed by inserting the needle into a suited portion of human skin and by moving the piston manually or by pressing a release button of an automatic injection device. Automatic injection devices may comprise an electronic dose dial mechanism to automatically set a dose.

The reusable or semi-reusable delivery device usually comprise a housing accommodating a dose and dispensing mechanism and a reservoir holder adapted to hold a reservoir, e.g. a cartridge or a syringe, in place relative to the housing.

Common devices have a reservoir holder that can be releasable attached to the dispensing mechanism to replace a cartridge. The reservoir holder may be coupled to the dispensing mechanism, for example, by a bayonet connection or by a thread. The user can remove the reservoir holder and load a new cartridge into the reservoir holder and subsequently attach the holder again to the dispensing mechanism.

Alternative concepts are known that include a front-loaded device which has a reservoir holder that remains permanently connected to the drive mechanism. In those concepts the user loads the cartridge into the reservoir holder from a distal end and into a distal opening in the reservoir holder. The cartridge may include a collar or cover that allows to couple releasably the cartridge to the distal end of the reservoir holder.

For example, WO 2015/165992 A1 discloses an electronic injection pen comprising a controller and a front-loading cartridge holder. To load a cartridge, the user rotates an actuation sleeve to a loading position, inserts the cartridge until it seats against fingers of a bias member and then rotates the sleeve back. This retracts the cartridge, actuates a cartridge switch, and signals the controller that a cartridge has been inserted and that the holder is closed.

WO 2014/187813 A1 discloses a front-loaded injection device comprising a cartridge holder with flexible arms that have gripping shoulders to hold a cartridge in place. The flexible arms are actuated by an outer tubular sleeve which is actuate by a rotation of an outer tube member and the tubular sleeve moves the gripping shoulders between open and closed positions to load and secure the cartridge.

Such known approaches often require a complex and often bulky rotation mechanism to lock or unlock the cartridge to the cartridge holder.

### DESCRIPTION OF THE INVENTION

It is an objective of the invention to provide a simple yet compact coupling mechanism for coupling a cartridge to a reusable injection device.

This objective is achieved by a dose and dispensing mechanism according to the independent claims. Preferred embodiments are evident from the dependent claims.

The present invention relates to a drug delivery device, and in particular an injection device, for dispensing a liquid drug from a reservoir through an injection needle. The delivery device comprises
- a reservoir holder defining a longitudinal axis (L) and adapted to hold the reservoir in place relative to a dispensing mechanism of the delivery device. The reservoir holder is connected to the dispensing mechanism at a proximal end or end portion and includes a holder connecting portion at a distal end or in a distal end portion;
- a collar comprising a fastening element fastening the collar non-releasably to a neck portion of the reservoir, in particular to a neck portion of a cartridge, wherein the collar includes a collar connecting portion, preferably at a proximal end, adapted to be releasably coupled to the holder connecting portion;
- a coupling member, a rotatable plunger rod and a revers lock. The coupling member can be in a reset position and in a distinct drive position. When the coupling member is in the reset position the plunger rod is decoupled from the reverse lock and the plunger rod can be rotated in a dispensing direction and in a opposite reset direction.
When the coupling member is in the drive position the plunger rod is coupled to the reverse lock and can be rotated exclusively in dispensing direction and is prevented from being rotated in the opposite reset direction.

The delivery device further includes a lock member arranged in the reservoir holder and extending between the distal end and the proximal end of the reservoir holder. The lock member can cooperate with or engage the collar at the distal end. Furthermore, the lock member can cooperate with or engage the coupling member at the proximal end of the lock member.

When the collar is attached to the reservoir holder by an attachment movement the lock member is moved relative to the reservoir holder and is adapted to move the coupling member from the reset position to the drive position during the attachment movement.

According to the present invention the reservoir holder includes at least one lock member that extends from a distal end to a proximal end of the reservoir holder. Hence, the lock member bridges the length of the reservoir holder along the longitudinal axis. When the user attaches the collar with the cartridge to the delivery device the user has to attach the collar by an attachment movement, e.g. by a rotational, a linear, a screw movement or a combination thereof. During this attachment movement the lock member is moved from a distal position towards a proximal position. As the lock member cooperates with its proximal end with a coupling member of a reset mechanism of the delivery device the lock member can move the coupling member from a reset position into a drive position. That means by the attachment movement the delivery device is switched from a reset state (in which the plunger rod can be pulled back) to a drive state (in which the plunger rod is coupled to the reverse lock and the delivery device is ready for an administration).

That means the lock member allows to bridge the length of the reservoir holder along the longitudinal axis and to switch the delivery device from a reset state into a drive state only by the attachment movement of the collar at the distal end of the reservoir holder (and thus at the distal end portion of the delivery device).

Accordingly, a detachment movement when the user removes the collar from the distal end of the reservoir holder may move the lock member, preferably in distal direction, and thereby may cause a movement of coupling member from the drive position to the reset position.

A rotation or additional user action to switch the device from a reset state to a drive state as in prior art approaches is not required. A complex and bulky rotational switch mechanism can be avoided.

The reservoir holder is preferably adapted to fully or partly envelop the reservoir and to hold the reservoir in place. In particular, the reservoir holder is adapted to hold the reservoir such that it cannot move relative to the dispensing mechanism and in particular to a dispensing mechanism housing.

The reservoir holder is preferably fixedly and non-releasably connected to a dose and dispensing mechanism, and preferably to the dispensing mechanism housing. The housing is preferably sleeve-shaped and adapted to accommodate the dispensing mechanism components. In this case, the reservoir holder is preferable fixedly (non-releasably and permanently) connected to the dispensing mechanism. That means the reservoir holder cannot be disconnected from the housing by the user. In particular, the reservoir holder may not be disconnectable from the housing without dedicated tool or without destruction.

Alternatively, the reservoir holder may be integrally formed (as a monolithic part) in a housing which accommodates the dispensing mechanism. That means a (preferably proximal) part of the reservoir holder is a housing adapted to accommodate components of the dispensing mechanism. The dispensing mechanism may include, for example, a dose setting member, a dispensing button or a manual or automatic drive member such as a spring.

The drug delivery device may be a reusable, a disposable, a semi-disposable (also named as semi-reusable) delivery device. The reservoir together with the collar are preferably disposed after use or when the reservoir is empty. The dispensing mechanism and the reservoir holder are preferably reusable and can be attached to a new reservoir via a collar. The drug delivery device is preferably an injection device, in particular a pen-shaped injection device.

The reservoir may be a container, cartridge (one or two chamber) adapted to contain the drug. The cartridge may be a non-refillable cartridge for single use. The cartridge includes a needle mounting portion where a needle assembly can be releasably attached to. Alternatively, the reservoir may be a prefilled syringe having a needle or cannula that is permanently attached to a first end of a syringe barrel that is sealed at the opposing end by the plunger.

The collar is adapted to fixedly connected to the reservoir at the manufacturer site or during an assembly. That means the collar is not adapted to be removed from the reservoir by the user. The collar along with the reservoir can be attached to the distal end of the reservoir holder.

The coupling member is adapted to switch the device between a reset state (reset position coupling member) and a drive state (drive position coupling member). In the reset state or reset position of coupling member the plunger rod can be moved in both direction (dispensing and counter direction) to reset the device and in particular to retract the plunger rod towards a start position to prepare the delivery device for a new injection. In the drive state or drive position of coupling member the plunger rod is coupled to the drive train and hence to a reverse lock that enables a rotation of the plunger rod in dispensing direction to dispense a dose but prevents a rotation of the plunger rod in counter reset direction.

The coupling member can be switched between the two position by a switch movement, for example a linear, rotational or screw movement. As the lock member can cooperate with the coupling member (e.g. by contacting or coupling) a lock member movement (linear, rotational, screw movement or combination) causes a movement of the coupling member between the reset position and the drive position.

Preferably, the coupling member is in the reset position when the lock member is in a distal position (collar not attached to reservoir holder) and the coupling member is in the drive position when the lock member is in a proximal position (collar attached to reservoir holder).

The coupling member may cause the plunger rod to engage the reverse lock, for example by spline engagement, by teeth or force-fit.

The lock member extends along the reservoir holder. By way of example, the lock member may be bar-shaped, sleeve-shaped or rod-shaped. The lock member may be arranged, for example, inside the reservoir holder or guided on an outer surface of the reservoir holder.

In the present context, the terms "substance", "drug", "medicament" and "medication" are to be understood to include any flowable medical formulation suitable for controlled administration through a means such as, for example, a cannula or a hollow needle, and comprises a liquid, a solution, a gel or a fine suspension containing one or more medical active ingredients. A medicament can be a composition comprising a single active ingredient or a pre-mixed or co-formulated composition with more than one active ingredient present in a single container. Medication includes drugs such as peptides (e.g., insulin, insulin-containing drugs, GLP-1 containing drugs or derived or analogous preparations), proteins and hormones, active ingredients derived from, or harvested by, biological sources, active ingredients based on hormones or genes, nutritional formulations, enzymes and other substances in both solid (suspended) or liquid form but also polysaccharides, vaccines, DNA, RNA, oligonucleotides, antibodies or parts of antibodies but also appropriate basic, auxiliary and carrier substances

The term "distal" is meant to refer to the direction or the end of the drug delivery device carrying an injection needle or an injection cannula, whereas the term "proximal" is meant to refer to the opposite direction or end pointing away from the needle or cannula.

The term "injection device" or "injector" refers to a device that is removed from the injection site after each medication event or drug delivery process, whereas the term "infusion system" refers to a device with a cannula or needle that remains in the skin of the patient for a prolonged period of time, for example, several hours.

The reservoir holder preferably includes a longitudinal guiding element allowing a movement of the lock member along the longitudinal axis and preventing a rotational movement of the lock member. The guiding element may be, for example, a groove, an axial recess, a rail or an axially extending cam. The guiding element may be integrally formed in the reservoir holder. The lock member may have a corresponding counter guiding element such as a groove, recess or cam.

Alternatively, the lock may be allowed to be rotated or screwed relative to the reservoir holder and may have a corresponding rotational guiding or thread element. **In** this case the lock member switches the coupling member between the reset position and the drive position by a rotational or screw movement.

In a preferred embodiment the attachment movement includes a rotational movement and the collar includes a guiding curve adapted to interact with a distal end of the lock member such that the rotational movement of the collar relative to the reservoir holder forces the lock member to move along the longitudinal direction. That means the guiding curve abuts or contacts the distal end of the lock member and thereby forces the lock member to move, preferably in proximal direction, to move the coupling member from the reset position into the drive position.

In a preferred the distal end of the lock member includes a first sloped surface and the guiding curve includes a second sloped surface such that upon contact the first and second sloped surfaces slide along each other thereby transferring the rotational movement of the collar into a movement of the lock member. The sloped surfaces may facilitate the transfer of the rotational movement of the collar to the lock member.

Alternatively, the collar does not include a guiding curve but may have, for example, a thread adapted to be in threaded engagement with the distal end of the lock member.

Preferably, guiding curve in the collar includes a first depression and the distal end of the lock member, in particular a tip, is adapted to fit into the first depression such that the lock member is in a stable position when the collar is fully attached to the reservoir holder. That means when the lock member is located inside the first depression the lock member cannot be moved out of the current position without extra force and thus remains in a stable position. Preferably, the lock member is positioned inside the first depression when the lock member has been moved proximally and is in a proximal position and the coupling member is in a drive position. The first depression thus ensures that the coupling member is not moved unintentionally out of its drive position (and the delivery device is not switched unintentionally out of the drive state).

The guiding curve may further comprise a second depression which is located distally to the first depression. During the attachment when the collar is rotated the distal end of the lock member is preferably moved from the second depression into the first depression.

The lock member is preferably positioned inside the second depression when the lock member is in a distal position and the coupling member is in the reset position. The second depression thus ensures that the coupling member is not moved unintentionally out of its reset position (and the delivery device is not switched unintentionally out of the reset state).

Preferably, the lock member is bar-shaped and comprises an arrowhead-shaped tip at the distal end. The bar-shaped lock member allows for a compact design. The tip may be adapted to cooperate with the collar such that during the attachment movement the collar cooperates or interacts with the tip, preferably with the guiding curve, if any, thereby moving the lock member to switch the coupling member between the reset position and the drive position.

Alternatively, the lock member may be sleeve-shaped or nut-shaped.

The reservoir holder is preferably fixedly connected to a housing of the dispensing mechanism such that the reservoir holder does not move during the attachment movement of the collar to the reservoir holder. That means the coupling member is exclusively swithed between the reset position and the drive position by the lock member. The reservoir holder is fixed and is not movable relative to the dispensing mechanism or/and a dispensing mechanism housing.

In a preferred embodiment holder connecting portion includes a cam or a L-shaped groove adapted to accommodate the cam and the collar connecting portion includes the other of the cam and the groove to provide a releasable bayonet connection between the collar and the reservoir holder. The bayonet connection provides a safe and reliable connection between the collar with the reservoir and the reservoir holder. Furthermore, the user can easily connect or release the bayonet connection which contributes to enhanced usability.

Preferably, the attachment movement for connecting the bayonet connection includes an axial movement and a subsequent rotational movement. Before the rotational movement the lock member is preferably in a distal position and after the rotational movement the lock member is preferably in a proximal position relative to the reservoir holder. That means during the attachment movement the lock member is shifted proximally thereby moving the coupling member from the reset position into the drive position.

In a preferred embodiment the collar connecting portion comprises a coding feature and the holder connecting portion includes a counter coding feature. The collar may be held at the distal end of reservoir holder only if the coding feature matches the counter coding feature.

The coding feature and the counter coding feature prevent the user from connecting an inappropriate or wrong or non-compatible (disposable) reservoir with the collar to a (reusable) housing of the dose and dispensing mechanism of the delivery device. That means only a specific, correct and compatible reservoir with a corresponding collar can be attached to the reservoir holder. The coding feature and the counter coding feature may be customized and configured to provide a definite (unique) coding for a particular collar with a reservoir, e.g. the coding may be unique for a specific drug or customer.

The coding feature and the counter coding feature exclusively match each other. That means if the coding feature does not match the counter coding feature the reservoir with the collar cannot be attached or connected to the reservoir holder by the user and thus cannot be connected to dispensing mechanism. That means the coding feature and the counter coding feature provide an exclusive, releasable and reliable form-fit connection.

The coding feature and the counter coding feature may be, for example, a protrusion and a groove adapted to accommodate the protrusion or a rib, a cam, a knob or a lobe and a corresponding slot, recess, keyway or the coding feature may be a non-standard customized thread including a coding thread part and a counter coding thread part exclusively matching each other.

To be more explicit a reservoir with a first collar including a first coding feature matches exclusively a first counter coding feature such that the first reservoir with the first coding collar may be held in the reservoir holder if and only if the first counter coding feature matches the first coding feature.

A reservoir with a second collar having a second coding feature different than the first coding feature exclusively matches a second counter coding feature different than first counter coding feature such that the reservoir with the second coding collar may be held in the reservoir holder if and only if the second counter coding feature matches the second coding feature.

For reason of clarity, the term "exclusively" means that only the first coding feature matches the first counter coding feature and the second coding feature matches only the second counter coding feature. That means the reservoir with the second collar and second coding feature does not match the first counter coding feature and thus the reservoir cannot be held in the reservoir holder.

Preferably, the reservoir holder comprises a coding ring comprising a fastening portion to attach (to fasten) the coding ring non-releasably to a distal end of a sleeve of the reservoir holder and the coding ring further comprises the holder connecting portion. Thus, the reservoir holder is an assembly comprising the coding ring and a holder sleeve.

In embodiments without coding ring the collar along with the reservoir is directly attached and mounted to a distal end of the holder sleeve (distal end of the reservoir holder). That means the holder connecting portion is arranged on a distal end of the holder sleeve.

In embodiments with the coding ring, the collar along with the reservoir is attached to a distal portion of the coding ring. That means the holder connecting portion is arranged on the coding ring. The coding ring in turn is non-releasably connected (e.g. by a snap-fit connection) to the holder sleeve.

In case the collar and the reservoir holder feature a coding as described above, a connecting portion of the ring includes the counter coding feature. A specific or unique counter ring coding feature can be chosen by the manufacturer or distributor to allow only a specific collar and reservoir to be attached to dose and dispensing mechanism via coding ring by the user.

As the ring is a separate part of the reservoir holder the connecting portion (and if any, the counter coding portion) can easily be adapted or customized. Unlike prior art approaches where the whole reservoir holder has to be replaced for a change or replace of the connecting portion only the ring can be changed or replaced. That means the volume and/or number of (plastic) parts to be replaced when changing the connecting portion and/or the coding is reduced which is cost-effective and is advantageous from an environmental view.

The connection between the reservoir holder and the ring is preferably a snap-fit connection which is established during a final assembly at the manufacturer site. The snap-fit connection is not intended to be released by the user and may be designed such that the release of the snap-fit connection requires a dedicated tool. The snap-fit connection allows an easy and reliable connection between the ring and the reservoir holder.

The counter coding feature of the ring may be customized and configured to provide a definite (unique) coding for a particular collar with a reservoir, e.g. for a specific drug or customer. Due to the snap-fit connection the ring with the counter coding feature may be easily exchanged to adapted to a specific collar and matching the coding feature.

The snap-fit connection may include, for example, a flexible snap arm on the ring or on the reservoir holder and a recess or opening for the arm on the other of the ring and reservoir holder.

Moreover, in a preferred embodiment the maximum outer diameter of the ring is equal or less than a maximum outer diameter of the holder sleeve of the reservoir holder. That means the holder sleeve diameter defines the outer most dimension of the device and the ring has the same outer diameter as the reservoir holder sleeve or has a smaller diameter as the reservoir holder sleeve. That means the ring does not enlarge the outer dimension of the device.

In a preferred embodiment the collar connecting portion includes at least three coding features or/and the holder connecting portion includes at least three counter coding features, wherein the at least three coding features or the at least three counter coding features are respectively identical and spaced apart in a circumferential direction such that the collar connecting portion and the holder connecting portion can be connected to each other in at least three different rotational positions.

The at least three coding features or/and the at least three counter coding features are preferably spaced apart in a circumferential direction such that the collar and the reservoir holder can be connected to each other in at least two or three different rotational positions, respectively. That improves usability as the reservoir can be connected in at least three different rotational orientation.

Alternatively, or in addition, the collar and/or the reservoir holder may include coding features or counter coding features respectively at different positions on the collar or reservoir holder. By way of example, the collar may include a first coding feature on an outer rim and a second coding feature on an inner or outer surface of the collar. Likewise, the reservoir holder may include a first counter coding feature on a proximal end and a second counter coding feature on inner surface of an opening or on an outer surface of the reservoir holder or of the coding ring, if any.

Preferably, the coding feature is a protrusion or a recess adapted to accommodate the protrusion in a connected state and wherein the counter coding feature is the other of the protrusion and the recess.

The protrusion provides a safe and reliably coding as an attachment and/or connection of collar to the reservoir holder can be reliably prevented when the protrusion does not match the recess.

Alternatively, the coding feature may be a knob extending along a circumference or it may be thread.

In a preferred embodiment the holder connecting portion includes a cam or a L-shaped groove adapted to accommodate the cam. The collar connecting portion preferably includes the other of the cam and the groove to provide a releasable bayonet connection. The bayonet connection provides a safe and reliable connection between the collar with the reservoir and the reservoir holder. Furthermore, the user can easily connect or release the bayonet connection which contributes to an enhanced usability.

In a preferred embodiment the cam or the groove includes a retaining element to hold the cam in an end position within the L-shaped groove when the bayonet connection is connected. The retaining element is further adapted to increase a breakaway force required to release the bayonet connection.

The breakaway force is the effort or force required to initially move the cam out of its end position within the L-shaped groove and thus to start the release and decoupling of the bayonet connection.

The retaining element ensures that the user needs an increase effort for moving the cam out of the end position within the L-shaped groove. Due to the retaining element an unintentional release of the bayonet connection can be prevented. By way of example, the retaining element may be a bulge, ledge, protrusion, narrowing section or a nose adapted to retain the cam.

**In** a further preferred embodiment, a first coding feature of a first collar matches a first counter coding features of a first reservoir holder (first matching pair). A second coding feature of a second collar different from the first coding feature preferably matches a second counter coding feature of a second reservoir holder different from the first counter coding feature (second matching pair). The first collar and first reservoir holder both have the same colour and the second collar and the second reservoir holder both have the same colour which is different from the colour of the first collar and first reservoir holder.

The colour coding may support and help the user to find a compatible and matching reservoir with the collar that matches and can be attached to a corresponding reservoir holder. Additionally, or instead of the colour the collar and the reservoir holder may include further markers or coding features to show the user which collar with reservoir matches to a specific reservoir holder. By way of example such markers may be a graphic structure a printed or/and three-dimensional patterns or signs, characters, numbers, letters, words or phrases.

The collar preferably comprises an arm-shaped engaging element adapted to engage the neck portion of the reservoir and preventing a movement of the reservoir relative to the collar along the longitudinal axis. The arm-shaped engaging element provides a simple and reliable fastening to connect (fasten) the collar to the reservoir such that any movement along the longitudinal axis between the collar and the reservoir is reliably prevented.

The arm-shaped element is preferably integrally formed in the collar, in particular if the collar is an injection molded plastic part the arm-shaped element may be integrally formed and monolithic with the rest of the collar.

Preferably, a proximal portion of the reservoir holder is fixedly, non-releasably and permanently connected to a distal end portion of a housing of the dispensing mechanism. The reservoir holder is not intended to be disconnected and/or removed from the housing by the user. The reservoir holder is preferably connected to the housing by the manufacturer, for example, by a non-releasable snap-fit connection.

In this embodiment, the reservoir holder has preferably approximatively the length of the reservoir. That means the reservoir holder completely envelops the reservoir and is adapted to hold the reservoir in place relative to the housing which accommodates the dose and dispensing mechanism of the drug delivery device. The reservoir holder is preferably sleeve-shaped.

In an alternative embodiment the reservoir holder may not be a separate part but may be integrally formed and monolithic with the housing. In this alternative embodiment the reservoir holder extends along an entire length of the dispensing mechanism along the longitudinal axis and the reservoir holder is adapted to accommodate the reservoir in a distal portion and is further adapted to envelop the dispensing mechanism in a proximal portion of the reservoir holder. The reservoir holder is in this embodiment works thus also as a housing for the dose and dispensing mechanism. The reservoir holder is preferably the outer most part of the drug delivery device.

Therefore, the function of holding the reservoir in place and housing the dose and dispensing mechanism is implemented in one single part which reduces number of delivery device components and the complexity of the device.

The invention relates further to a method for connecting a collar to a reservoir holder of a drug delivery device. The method comprises the steps of
a) Attaching a collar connecting portion of the collar to a distal end of the reservoir holder, the collar being non-releasably connected to a reservoir containing a liquid drug;
b) Moving, for the attachment, the collar relative to the reservoir holder;
c) Moving a lock member relative to the reservoir holder, by the collar attachment movement, wherein the lock member extends between a distal end and a proximal end of the reservoir holder;
d) Abutting and moving a coupling member of a dispensing mechanism of the drug delivery device, by a proximal end of the lock member during the lock member movement, from a reset position into a drive position;
e) Coupling a plunger rod, by the coupling member movement, with a reverse lock such that the plunger rod is exclusively movable in the dispensing direction and is prevented from being moved in a reset direction by the reverse lock when the coupling member is in the drive position.

### BRIEF DESCRIPTION OF THE DRAWINGS

The subject matter of the invention will be explained in more detail in the following text with reference to preferred exemplary embodiments which are illustrated in the attached drawings, in which:
- Fig.1: depicts an exploded view of a reusable injection pen according to the present invention;
- Fig.2: depicts a sectional view of a distal end portion of the injection pen;
- Fig.3: depicts a sectional view of middle portion of the dose and dispensing mechanism of the injection pen;
- Fig.4: depicts a sectional view of the injection pen in a reset state without cartridge and collar and
- Fig. 5: depicts a sectional view of the injection pen in a dispensing state.

The reference symbols used in the drawings, and their primary meanings, are listed in summary form in the list of designations. In principle, identical parts are provided with the same reference symbols in the figures.

### DETAILED DESCRIPTION OF PREFERRED EMBODIMENTS

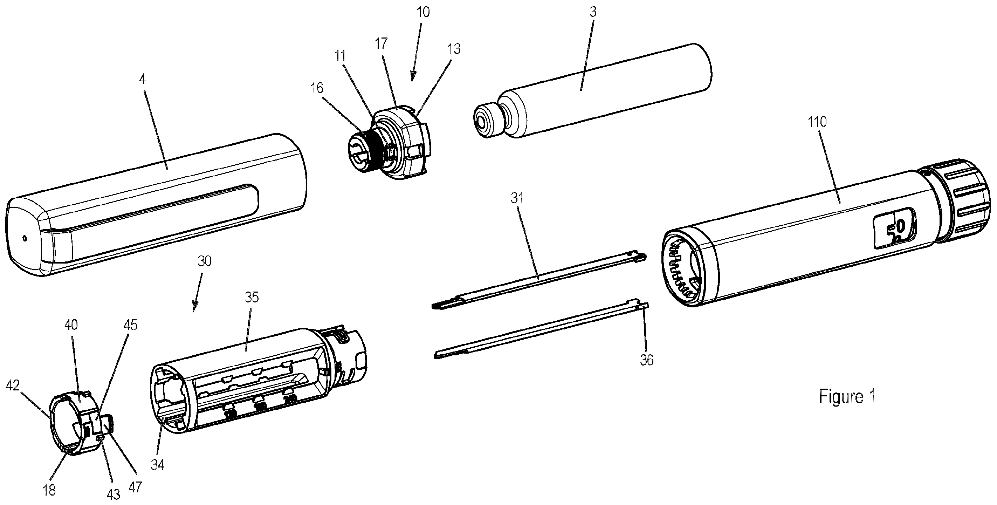

Figures 1 to 5 depict a drug delivery device in form of a reusable injection pen 1 according to the present invention. The injection pen includes a reset mechanism which enables the injection pen to switch between a dispensing state and a reset state as will be described in detail in the following.
Figure 1 depicts a perspective view of the components of the injection pen 1. The pen includes a cap 4, a coding collar 10, a cartridge 3, a cartridge holder 30, two lock bars 31 and a housing 110 accommodating a dose and dispensing mechanism. The cartridge holder 30 comprises a holder sleeve 35 and a coding ring 40.

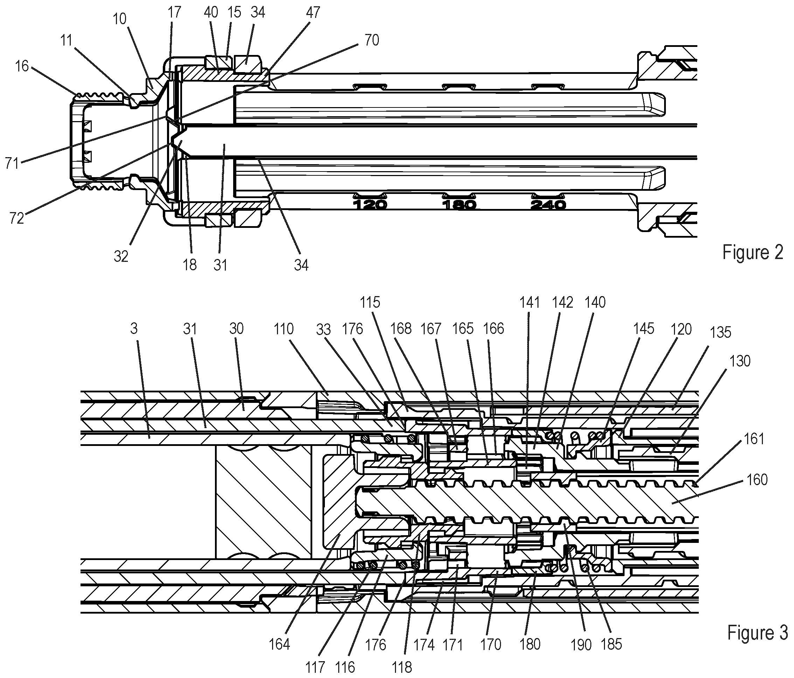

Figure 2 depicts sectional view of a distal portion of the cartridge holder with the holder sleeve and, the collar 10 and the coding ring 40 without cartridge.

The coding collar 10 is connected to a neck portion of the cartridge 3 by two oppositely arranged snap arms 11 (figure 1 and 2) which engage and clamp the cartridge neck portion. The cartridge 3 is therefore fixedly and non-releasably connected to the collar 10. The collar 10 is mounted to the cartridge at the manufacturer site and is not intended to be removed from the cartridge 3 by the user.

The snap arms 11 are located between a distal needle mounting portion 16 which is adapted to be releasably attached to a needle assembly with an injection needle and a sleeve or ring-shaped proximal portion 17 with a larger diameter than the needle mounting portion 16, see figures 1 and 2.

The coding ring 40 includes a holder connection portion on a distal end including the L-shaped grooves 45 (figure 1). The collar includes a collar connecting portion in form of the cams 15 adapted to engage the L-shaped grooves for the bayonet connection between the collar 10 and the ring. The ring 40 includes in total four L-shaped grooves 45 along the circumference spaced apart from each other in an angle of 90°. Therefore, the collar 10 can be connected to the ring 40 in four different rotational orientations.

Next to each L-shaped groove 45 and thus in total four coding protrusions 43 are positioned on an outside of the ring. The protrusions 43 exclusively match the recesses 13 in the collar 10 as described above. Additionally, the ring 40 comprises on an inside of a distal end rim four coding recesses 42 which are 90° spaced apart from each other along the circumference. The coding recesses 42 are designed to match the coding protrusion 12 of the collar 10.

Inside the opening the collar 10 includes two oppositely arranged cams 15 (figure 2) adapted to be inserted into the L-shaped grooves 45 (figure 1) in the coding ring 40 to connect a bayonet connection between the collar 10 and the ring 40. On an outer surface the ring further includes four coding protrusions 43. In an end rim (proximally faced end surface) the collar 10 comprises four coding recesses 13 (figure 1) which have an elongated form in the circumferential direction and which are evenly distributed along the circumference.

With the above-described coding collar 10 and the coding ring 40 a coding and counter coding feature is implemented. A first coding is provided by the cams 15 which are designed such that the cams exclusively match the L-shaped grooves 45 of the ring. The grooves 45 thus acts as counter coding features. A second coding is provided by coding protrusions (not shown) on the inside of the collar 10. These protrusions exclusively match counter recesses 42 in the ring (figure 1). The collar coding recesses 13 on the proximal end of the collar provide a third coding as counter protrusions 43 of the ring are designed to exclusively match the recesses 13 of the collar 10. All recesses 13, 42 have an elongated form and are designed such that the protrusions 12, 43 can travel within the recesses during relative rotation between the collar 10 and the ring 40 when the bayonet connection is coupled or released.

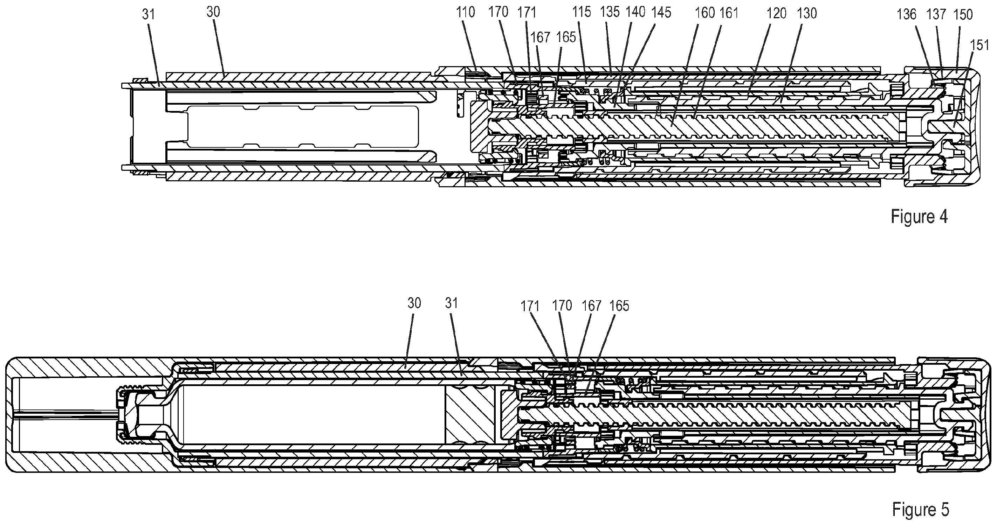

The coding ring 40 comprises two oppositely positioned snap arms 47 for a snap fit connection with a distal end portion of the reservoir holder 30 as shown in figure 1 and 2. The ring 40 is thus immovably held to the reservoir holder 30 and is not intended to be decoupled and separated from the reservoir holder by the user after assembly. The ring 40 along with the holder sleeve 35 provide an opening to accommodate the cartridge 3 in an attached state as shown in figure 5.

The coding collar 10 which matches a coding ring 40 (matching combination) may have the same colour to support the user to connect a compatible and correct collar and cartridge with a corresponding ring of a reservoir holder.

Inside the cartridge holder are the two oppositely arranged lock bars 31 that can be actuated and moved to switch the injection pen between a reset state and a dispensing state as described in detail in the following.

Referring to figure 2 it can be seen that the collar 10 includes a guiding curve 70 adapted to cooperate with the bars 31 and thus forming a cam control. The guiding curve 70 is formed on a proximally oriented surface inside the collar 10. As shown in figure 2, the guiding curve 70 has two depressions 71, 72 separated from each other by a tip pointing in proximal direction. The two depressions 71, 72 form rest positions for a distal tip 32 of each lock bar 31. Each tip 32 has an arrow-head shape which fits into the depressions 71, 72 such that the lock bars 31 can be in a stable position.

The coding ring 40 and the cartridge holder 30 include longitudinal guiding grooves 18, 34 for the lock bars 31 such that the lock bars 31 can only be moved along the longitudinal direction and are prevented from being rotated or deflected. Each lock bar 31 includes an end stop protrusion 36 (figure 1) which prevents the lock bars 31 to drop out.

The guiding curve 70 inside the collar and the corresponding longitudinal guiding grooves 18, 34 are oppositely arranged such that the lock bars 31 are 180° separated in a circumferential direction.

In the following paragraphs the components of the dose and dispensing mechanism are described. Subsequently, the function is described.

As shown in figures 3 to 5, the housing 110 is the outermost part and an insert sleeve 115 is fixedly connected to the housing 110 and comprises on its outside a thread engaging an inner thread of a scale drum or number sleeve 135 which is located coaxially outside the insert sleeve 115 and inside the housing. The scale drum 135 indicates the dose values to the user.

Coaxially inside the housing insert sleeve 115 a clutch sleeve 120 is located and splined to an inner surface of the insert sleeve 115 such that the clutch sleeve 120 is axially movable but prevented from being rotated relative to the insert sleeve 115 and the housing 110. The clutch sleeve 120 includes a thread segment on its inner surface engaging an outer thread of a dose sleeve 130 which is coaxially inside the clutch sleeve 120. On a proximal end the dose sleeve 130 is fixedly connected to a collar 136 which comprises cams 137 on its outside adapted to be releasably coupled to grooves inside a dose knob 150 (figure 4). The dose knob 150 can thus be rotationally and releasably coupled to the dose sleeve 130 via cams 137 and grooves. Due to its thin-walled shape the collar 136 can be deformed if a certain force threshold is exceeded. That means, for example, if the dose sleeve 130 with the collar 136 is rotationally blocked and if the user continues to apply a torque to the rotationally coupled dose knob the collar 136 may deform (e.g. deflect radially inwards) and the cams 137 may move relative to the grooves. The collar 136 thus provides an overload clutch that may preserve the mechanism from damage if the torque exceeds a threshold. The dose knob 150 further includes a cam-shaped bearing 151 (figure 4) providing a support for the dose knob 150 such that the latter can be rotated relative to the dose sleeve 130 in a decoupled state.

The dose sleeve 130 is further rotationally connected to the scale drum 135 via spline engagement but is movable axially relative to the scale drum.

Inside the dose sleeve 130 a drive sleeve 140 is coaxially positioned and splined to the dose sleeve 130 such that a relative rotation is prevented but a relative axial movement between the drive sleeve 140 and the dose sleeve 130 is allowed. The drive sleeve 140 is axially connected to the clutch sleeve 120 by a connecting disc 145 such that an axial movement of the clutch sleeve 120 is transferred to the drive sleeve 140 but a relative rotation is not transferred. The drive sleeve 140 is coupled to a plunger rod 160 via a drive nut. The plunger rod 160 with an outer thread 161 is arranged inside the drive sleeve 140 and the plunger rod 160 is in threaded connection with a bearing sleeve 118, which is fixedly connected to the housing 110. A distal end of the plunger rod is rotatably connected to a flange 164 which abuts a proximal face of a piston inside the cartridge 3. Furthermore, the flange 164 provides for a non-rotating contact surface for manual reset or proximal retraction movement of the plunger rod upon cartridge replacement.

The drive sleeve 140 includes in a distal portion a coupling part 142 including splines 141 on an inner surface adapted to engage corresponding splines or grooves 166 on an outside of the sleeve-shaped drive nut 165 surrounding the plunger rod 160. The drive nut 165 is axially coupled to the bearing sleeve 118 and thus to the housing but is allowed to rotate relatively thereto. Moreover, the drive nut 165 is splined to the plunger rod 160 and hence rotationally coupled to it.

The clutch sleeve 120 and drive sleeve 140 are axially movable relative to the housing. That is, during dose setting the drive sleeve 140 can be in a proximal dose setting position in which the spline engagement to the drive nut 165 is released (figure 4) allowing a rotation of the drive sleeve 140 relative to the drive nut 165 and thus a rotation of the drive sleeve 140 is not transferred to the plunger rod 160. The drive sleeve 140 can be further in a distal dispensing position in which the spline engagement is established and a rotation of the drive sleeve 140 is transferred to the plunger rod 160 via drive nut 165.

Radially outside of the coupling part 142 and releasably and rotationally coupled to the drive sleeve a dosing click ring 180 is located comprising on a distal front surface axially extending dosing click teeth engaging axially extending counter click teeth. The latter are arranged on a proximal front surface of a ratchet sleeve 170. The dosing click ring 180 is biased in distal direction by a reset spring 185 which abuts on a distal side onto the dosing click ring 180 (figure 3) and on a proximal side on the connecting disc 145. Therefore, the dosing click teeth are pressed onto the counter click teeth. The drive sleeve 140 comprises in a distal portion radially extending protrusions adapted to be engaged in corresponding grooves in the dosing click ring 180 to rotationally couple the drive sleeve 140 to the dosing click ring 180.

The ratchet sleeve 170 is located outside the bearing sleeve 118 and is rotationally fixed to the housing 110 but axially moveable relative thereto for a device reset operation. Counter dosing click teeth are arranged on an outer circle on a proximally oriented front face of the ratchet sleeve 170. On an inner wall of the ratchet sleeve 170 are circularly arranged and radially inwards extending counter dispensing click teeth 171 adapted to engage a dispensing click tooth 168 of a radially extending dispensing click arm 167 of the drive nut 165. The drive nut 165 includes in total two oppositely arranged flexible radial click arms 167 that engage the counter dispensing click teeth 171. The teeth 168 of the arms 167 and the counter dispensing click teeth 171 are adapted to slide along each other in a first dispensing direction but prevent a relative rotation in the counter reset direction.

On its outside the ratchet sleeve 170 comprises two oppositely arranged guiding rails 174. The rails 174 rotationally couple the ratchet sleeve 170 to the insert sleeve 115. Moreover, the ratchet sleeve has a distally oriented contact surface 176 (figure 3) adapted to be contacted by the lock bars 31.

In an attached position and dispensing state a proximal end of each the lock bar 31 presses on the distal contact surface 176 of the ratchet sleeve 170 thereby holding the ratchet sleeve in place as the sleeve is biased in distal direction by the spring 185. The ratchet sleeve 170 further comprises two oppositely arranged and axially extending cams which rotationally couple the ratchet sleeve 170 to a linear guide of the housing 110 via insert sleeve 115. On its inside the ratchet sleeve 170 includes the radial counter dispening teeth (ratchet teeth) 171 and further includes four radially inward extending protrusions which act as guiding means to guide the drive sleeve 140 to ensure that the protrusions and grooves correctly engage each other when the drive sleeve 140 is about to couple with the dosing click ring 180 during proximal retraction of the drive sleeve.

A stop nut 190 is in treaded engagement with the outer plunger rod thread 161. The stop nut 190 comprises a stop surface oriented in a plane parallel to the longitudinal axis and adapted to abut a counter stop surface of an end portion of the plunger rod 160. The outer thread 161 may have a thread pitch that changes at the proximal end of the thread. In particular, the thread may include an end thread segment that has a larger thread pitch (larger thread angle) than the rest of the plunger rod thread 161. Due to that end thread segment the force acting on the plunger rod end portion can be reduced when the stop nut 190 abuts the stop surface.

The stop nut 190 further includes on its outside six circumferentially arranged cams that are guided in corresponding grooves on an inner surface of the drive sleeve 140. The stop nut 190 can thus move axially relative to the drive sleeve 140 but is prevented from being rotated relative thereto.

Distally to the bearing sleeve 118 a cartridge support 116 is located which is biased in distal direction by a cartridge spring 117 pushing the support 116 against a proximal end rim of the cartridge. The biased cartridge support 116 ensures that the cartridge is biased in distal direction and thus safely held in place within the cartridge holder 30.

To set a dose the user grasps the dose knob 150 and rotates it. The rotation and the torque are transferred to the dose sleeve 130 and to the scale drum 135 by the overload clutch (collar 136). Due to the threaded connection with the clutch sleeve 120, the dose sleeve 130, the collar 136, the scale drum 135 and the dose knob 150 are moved in proximal direction.

As the drive sleeve 140 is rotationally coupled to the dose sleeve 130 the drive sleeve 140 is rotated too together with the dosing click ring 180. Therefore, the dosing click teeth slide over the counter dosing click teeth of the ratchet sleeve 170 (which is not moved) thereby generating a click sound.

During dose setting the drive sleeve 140 is in its proximal dose setting position and hence not coupled to the drive nut 165 and thus the drive nut 165 and the plunger rod 160 are not rotated relative to the housing 110. The clutch sleeve 120 cannot rotate as it is splined to the housing 110. As the drive sleeve 140 is rotated relative to the plunger rod the stop nut 190 is screwed in proximal direction during dose setting.

To correct a dose the user can rotate the dose knob 150 in counter direction to decrease the dose and thus the dose sleeve 130 is screwed back into the housing.

Once the correct dose is set the user presses onto dose knob 150 and shifts the dose knob 150 relative to the collar 136 in distal direction. The axial travel of the dose knob 150 moves the grooves of the knob out of engagement with the cams 137 and thus rotationally decouples the dose sleeve 130 from the dose knob 150. Furthermore, the axial user force acts on the dose sleeve 130 via bearing 151 and the dose sleeve pushes the clutch sleeve 120 via threaded engagement distally. As the connecting disc 145 abuts on a ledge of the drive sleeve 140, the drive sleeve 140 is moved from its dose setting position distally into its dispensing position.

That means the drive sleeve 140 is moved distally relative to the dosing click ring 180 and thus the drive sleeve protrusions get out of engagement with the ring grooves and thus the drive sleeve rotationally decouples from the dosing click ring 180. The drive sleeve 140 is also moved relative to the drive nut 165 and the coupling portion 142 with the splines 141 (protrusions or grooves) are moved onto the splines 166 (protrusions or grooves) on an outer proximal coupling portion of the drive nut 165. The drive sleeve 140 is thus rotationally coupled to the drive nut 165 and thus to the plunger rod 160.

When the user further presses the dose knob 150 in distal direction the dose sleeve 130 is screwed back into the housing 110. The drive sleeve 140 is rotated in dispensing direction and the rotationally coupled drive nut 165 is rotated too which in turn is rotationally coupled to the plunger rod 160 and thus transfers the rotation to the plunger rod 160. As the latter is in threaded connection with the bearing sleeve 118 the plunger rod 160 is screwed in distal direction. This moves the piston inside the cartridge barrel in distal direction to dispense the liquid drug.

The rotation of the drive nut 165 causes flexible click arms 167 with the dispensing click teeth 168 to slide over the counter dispensing click teeth 171 of the clutch sleeve 170 and thereby generate a click sound during dose dispensing.

During dose dispensing the drive sleeve 140 rotates together with the plunger rod 160 and hence there is no relative rotation therebetween. Therefore, the stop nut 190 does not move relative to the plunger rod 160 and remains in its position.

In order to prepare the injection pen 1 for a new injection and to reset the dispensing mechanism the user rotates the collar 10 relative to the coding ring 40 and hence relative to the cartridge holder 30 and subsequently moves distally the collar 10 and cartridge 3 by a linear retraction movement to release the bayonet connection. That means the spring 185 can relax and the ratchet sleeve 170 and the lock bars 31 are moved distally relative to the cartridge holder 30.

The ratchet sleeve 170 can then move into a reset position and the dose and dispensing mechanism is then in a reset state as shown in figure 4. That means the reverse lock provided by the flexible click arms 167 with its teeth 168 and the dispensing click teeth 171 of the ratchet sleeve 170 are axially separated from each other and the plunger rod 160 can be pushed back into the housing 110 by the user. To that end, the user presses on the distal surface of the non-rotating flange 164 and forces the latter proximally, leading to reverse rotation of plunger rod 160 and drive nut 165. Alternatively, the plunger rod 160 is moved back by the cartridge when a user puts a new cartridge into the cartridge holder 30. In the reset position of the ratchet sleeve 170 the plunger rod is rotatable in dispensing and in counter reset direction.

The user then inserts a new cartridge 3 into the cartridge holder 30 and attaches the collar 10 to the coding ring 40. After a proximal linear movement, the collar 10 has to be rotated relative to the cartridge holder 30 to establish the bayonet connection. During this rotation the guiding curve 70 is moved relative to the tips 32 of the lock bars 31.

Tips 32 are in the depression 71 (figure 2) when the ring 40 axially shifted. After the subsequent rotation when the bayonet connection is closed the tips 32 move from depression 71 to depression 72. The depression 72 is located proximally to the depression 71.

During the rotational movement of the collar 10 and due to the sloped portions in the guiding curve 70 and the arrow-head shaped tips 32 the lock bars 31 are forced in proximal direction. That means a proximal end 33 (figure 3) of each lock bar 31 is thus pressed against the distal contact surface 176 of the ratchet sleeve 170 which is thereby moved proximally. That in turn means the ratchet sleeve is moved from its reset position into its drive position and the dispensing mechanism is then in a dispensing state. That means the ratchet sleeve counter dispensing click teeth 171 are brought into engagement with the dispensing click teeth 168 of the flexible click arms 167 and thus the reverse lock is enabled. The injection pen is then ready for dose setting and dose dispensing.

While the invention has been described in detail in the drawings and foregoing description, such description is to be considered illustrative or exemplary and not restrictive. Variations to the disclosed embodiments can be understood and effected by those skilled in the art and practising the claimed invention, from a study of the drawings, the disclosure, and the appended claims. **In** the claims, the word "comprising" does not exclude other elements or steps, and the indefinite article "a" or "an" does not exclude a plurality. The mere fact that certain elements or steps are recited in distinct claims does not indicate that a combination of these elements or steps cannot be used to advantage, specifically, in addition to the actual claim dependency, any further meaningful claim combination shall be considered disclosed.

**LIST OF DESIGNATIONS**

| | | | |
|---|---|---|---|
| 1 | injection pen | 70 | guiding curve |
| 3 | cartridge | 71 | depression |
| 4 | cap | 72 | depression |
| | | 110 | housing |
| 10 | coding collar | 115 | insert sleeve |
| 11 | snap arm | 116 | cartridge support |
| | | 117 | cartridge spring |
| 13 | coding recess | 118 | bearing sleeve |
| 15 | cam | 120 | clutch sleeve |
| 16 | needle mounting portion | 130 | dose sleeve |
| 17 | proximal portion | 135 | scale drum/number sleeve |
| 18 | guiding groove | 136 | collar |
| | | 137 | cams |
| 30 | cartridge holder | 140 | drive sleeve |
| 31 | lock bars | 141 | splines |
| 32 | tip | 142 | coupling part |
| 33 | proximal end | 145 | connecting disc |
| | | 150 | dose knob |
| 34 | guiding groove | 151 | bearing |
| 35 | holder sleeve | | |
| 36 | end stop protrusion | 160 | plunger rod |
| | | 161 | thread |
| 40 | coding ring | 164 | flange |
| 42 | coding recess | 165 | drive nut |
| 43 | coding protrusion | 166 | splines |
| | | 167 | click arm |
| 44 | axial groove section | 168 | dispensing click tooth |
| 45 | L-shaped groove | | |
| 46 | radial groove section | 170 | ratchet sleeve |
| 47 | snap arm | 171 | counter dispensing click teeth |
| 48 | retaining nose | 174 | guiding rails |
| | | 176 | contact surface |
| | | 180 | click ring |
| | | 182 | click teeth |
| | | 190 | stop nut |

## Claims

1. A drug delivery device (1) for dispensing a liquid drug from a reservoir (3), the delivery device (1) comprising
- a reservoir holder (30) defining a longitudinal axis (L) and adapted to hold the reservoir (3) in place relative to a dispensing mechanism, wherein the reservoir holder (30) has a proximal end which is connected to the dispensing mechanism and a distal end comprising a holder connecting portion (45);
- a collar (10) comprising a fastening element (11) fastening the collar (10) non-releasably to a neck portion of the reservoir (3), wherein the collar further comprises a collar connecting portion (15) adapted to be releasably coupled to the holder connecting portion (45);
- a coupling member (170), a rotatable plunger rod (160) and a reverse lock, wherein when the coupling member (170) is in a reset position the plunger rod (160) is decoupled from the reverse lock and the plunger rod can be rotated in a dispensing direction and in a opposite reset direction;
wherein when the coupling member (170) is in a drive position the plunger rod (160) is coupled to the reverse lock and can be rotated exclusively in dispensing direction and is prevented from being rotated in the opposite reset direction;
**characterized in that** the delivery device (1) further includes a lock member (31) extending between the distal end and the proximal end of the reservoir holder (30), wherein the lock member (31) can cooperate with the collar (10) at the distal end and with the coupling member (170) at the proximal end,
wherein the collar connecting portion (15) and the holder connecting portion (45) can be connected by an attachment movement to attach the collar (10) to the reservoir holder (30), wherein during the attachment movement the lock member (31) is moved by the collar (10) relative to the reservoir holder (30) and thereby the coupling member (170) is moved by the lock member (31) from the reset position to the drive position.

2. Drug delivery device (1) according to claim 1, wherein the reservoir holder (30) includes a longitudinal guiding element (34) allowing a movement of the lock member (31) along the longitudinal axis and preventing a rotational movement of the lock member (31).

3. Drug delivery device (1) according to claim 2, wherein the attachment movement includes a rotational movement and wherein the collar (10) comprises a guiding curve (70) adapted to cooperate with a distal end of the lock member (31) such that the rotational movement of the collar (10) relative to the reservoir holder (30) forces the lock member (31) to move along the longitudinal axis.

4. Drug delivery device (1) according to claim 3, wherein the guiding curve (70) comprises a depression (72) and the distal end of the lock member is adapted to fit into the depression (72) when the collar (10) is attached to the reservoir holder (30) such that the lock member (31) is in a stable position.

5. Drug delivery device (1) according to claim 4, wherein the guiding curve (70) further includes a second depression (71) which is located distally to the first depression (72) and wherein during the rotation of the collar the distal end of the lock member is moved from the second depression (71) into the first depression (72).

6. Drug delivery device (1) according to any of claims 1 to 5, wherein the lock member (31) is bar-shaped and the distal end is an arrowhead-shaped tip (32).

7. Drug delivery device (1) according to any of claims 1 to 6, wherein the reservoir holder (30) is fixedly connected to a housing (110) of the dispensing mechanism such that the reservoir holder (30) does not move during the attachment movement.

8. Drug delivery device (1) according to claim 1 to 7, wherein the holder connecting portion includes a cam (15) or a L-shaped groove (45) adapted to accommodate the cam and the collar connecting portion includes the other of the cam (15) and the groove (45) to provide a releasable bayonet connection between the collar (10) and the reservoir holder (30).

9. Drug delivery device (1) according to any of claims 1 to 8, wherein the collar connecting portion comprises a coding feature (13) and the holder connecting portion includes a counter coding feature (43) such that the collar (10) may be held at the distal end of reservoir holder (30) only if the coding feature (13) matches the counter coding feature (43).

10. Drug delivery device (1) according to claim 9, wherein the reservoir holder (30) comprises a coding ring (40) comprising a fastening portion (47) fastening the coding ring (40) non-releasably to the distal end of a holder sleeve (35) of the reservoir holder (30) and the coding ring (40) further comprises the holder connecting portion.

11. Drug delivery device (1) according claim 9 or 10, wherein the collar connecting portion includes at least three coding features (13) or the holder connecting portion includes at least three counter coding features (43), wherein the at least three coding features (13) or the at least three counter coding features (43) are respectively identical and spaced apart in a circumferential direction such that the collar connecting portion and the holder connecting portion can be connected to each other in at least three different rotational positions.

12. Drug delivery device (1) according to any of claims 9 to 12, wherein the coding feature (13) is a protrusion or a recess adapted to accommodate the protrusion when the collar connecting portion is connected to the holder connecting portion and wherein the counter coding feature (43) is the other of the protrusion and the recess.

13. Drug delivery device (1) according to any of claims 9 to 12, wherein a first coding feature of a first collar (10) matches a first counter coding features of a first reservoir holder (30) and wherein a second coding feature of a second collar different from the first coding feature matches a second counter coding feature of a second reservoir holder different from the first counter coding feature wherein the first collar (10) and first reservoir holder (30) both have the same colour and the second collar and the second reservoir holder both have the same colour which is different from the colour of the first collar (10) and first reservoir holder (30).

14. Drug delivery device (1) according to any of claims 1 to 13, wherein the fastening element of the collar (10) is a snap arm (11) adapted to engage the neck portion and preventing a movement of the reservoir (3) relative to the collar (10) in the longitudinal direction.

15. Method for connecting a collar (10) to a reservoir holder (30) of a drug delivery device (1), the method comprising the steps of
a) Attaching a collar connecting portion of the collar (10) to a distal end of the reservoir holder (30), the collar being non-releasably fastened to a reservoir (3) containing a liquid drug;
b) Moving, for the attachment, the collar (10) relative to the reservoir holder (30);
c) Moving a lock member (31) relative to the reservoir holder (30), by the collar attachment movement, wherein the lock member (31) extends between a distal end and a proximal end of the reservoir holder (30);
d) Abutting and moving a coupling member (170) of a dispensing mechanism of the drug delivery device (1), by a proximal end (33) of the lock member (31) during the lock member movement, from a reset position into a drive position;
e) Coupling a plunger rod (160), by the coupling member movement, with a reverse lock such that the plunger rod (160) is exclusively movable in the dispensing direction and is prevented from being moved in a reset direction by the reverse lock when the coupling member is in the drive position.
